# EUROPEAN PATENT APPLICATION

(11) **EP 4 374 775 A1**
(43) Date of publication of application: **29.05.2024**
(21) Application number: 22306619.2
(22) Date of filing: 26.10.2022
(51) Int. Cl.: A61B 5/00, A61B 5/145

(54) **OPEN FLOW CELL PROCESS AND SYSTEM FOR FABRICATING MICRONEEDLES**

(71) Applicant: PKvitality, 75001 Paris (FR)
(72) Inventor: PIERART, Luc, 94800 VILLEJUIF (FR); PEACOCK, Martin, ROYSTON, SG8 5HB (GB); TOWNSEND, Christopher, MITCHELDEAN, GL1 70D (GB)

(57) **Abstract**

The invention relates to a method comprising the steps of:
a) disposing an array (10) of raw microneedles (11) in front of a conductive element (12) so that the conductive element (12) is at equidistance of every raw microneedle (11) and not in contact with the tips of the raw microneedles (11);
b) immersing the array (10) of raw microneedle(s) (11) and the conductive element (12) in a solution (13) comprising an active element (14),
c) applying a potential (V) or a current (I) between the conductive element (12), forming a cathode, and at least one raw microneedle (11) of the array (10) of raw microneedles (11) forming an anode, causing an electrodeposition of a uniform and homogenous film comprising the active element (14) on the raw microneedles (11) of the anode.

## Description

### TECHNICAL FIELD

The present application relates to electrochemical sensors and in particular sensors for measuring a body analyte by means of microneedles. More precisely, the present application concerns a system and method for fabricating and/or functionalizing such microneedles for use in a device for measuring a body analyte.

### BACKGROUND OF THE INVENTION

Certain pathologies such as diabetes require daily monitoring of biochemical parameters of the human body, such as concentrations glucose containing in the interstitial liquid.

There are known devices with microneedles, which have the advantage of being less invasive that of conventional needles. Document WO 2020/025821 A1 discloses such a device.

These devices use amperometric measures for detecting the analyte so that the microneedles need to have chemical sensing capabilities. Indeed, for the detection of the glucose, the microneedles form several types of electrodes some of which having an enzyme deposited at the tips of the microneedles.

For the deposition of an element such as metal particle or an enzyme, it is possible to realize electrodeposition and/or polymerization for entrapping metal particle and/or enzyme onto the surface of the microneedles.

However, this fabrication of the microneedles is not homogenous especially when a large number of microneedles need to be manufactured.

### SUMMARY OF THE INVENTION

The present invention aims at improving the fabrication of microneedles for use in a device for measuring a body analyte such as glucose, lactate etc.

To this end, the invention concerns according to a first aspect, a method comprising the steps of:
a) disposing an array of raw microneedles in front of a conductive element so that the conductive element is at equidistance of every raw microneedle and not in contact with the tips of the raw microneedles;
b) immersing the array of raw microneedle(s) and the conductive element in a solution comprising an active element,
c) applying a potential or a current between the conductive element, forming a cathode, and at least one raw microneedle of the array of raw microneedles forming an anode, causing an electrodeposition of a uniform and homogenous film comprising the active element on the raw microneedles of the anode.

The invention according to the first aspect may comprise at least one of the following features, alone or in combination:
- an external reference electrode is disposed into the solution and not to be in contact with the raw microneedles and the conductive element, and not between the raw microneedles and the conductive element, the potential being also applied to the external reference electrode;
- the conductive element is a conductive grid or mesh plate or array of tips;
- the solution is a saline solution, the active element being metallic particles or an electroactive monomer precursor with a biochemical element;
- the tip of each raw microneedle is in front of the conductive element, each at a same distance of the conductive element, the distance being comprise between 0,1mm and 10mm;
- the potential (V) or current is an oxidizing potential or current and/or a reducing potential or current, the potential or current being a fixed potential or a variable potential said potential or current being applied using amperometric method and/or chronoamperometric method and/or potentiometric method and/or chronometric method and/or voltametric cycle.

The method according to the first aspect, can be used as a metallization step wherein the active element is conductive particles, the microneedles obtained at step c) are metallized microneedles adapted to be an electrode for an electrochemical sensor.

The method according to the first aspect can be used as a functionalizing step wherein the active element is an electroactive monomer precursor with a biochemical element, the microneedles obtained at step c) are functionalized microneedles adapted to be used in a working electrode for an electrochemical sensor.

The invention concerns according to a second aspect, a system comprising:
a receptacle configured to be filled with a solution comprising an active element,
at least one conductive element intended to be disposed in front of an array of raw microneedles and at equidistance of every raw microneedle and not in contact with the tips of the raw microneedles,
a power source configured to apply a potential or a current between the conductive element, forming a cathode, and at least one raw microneedle of an array of raw microneedles, forming an anode, the conductive element aiming at an electrodeposition of a uniform and homogenous film on each the raw microneedles of the anode when immersed in the solution and when the potential is applied, the film comprising the active element.

The invention according to the second aspect may comprise one of the following features alone or in combination:
- it comprises a holder for holding at least one conductive element and at least one an array of raw microneedles in front of the conductive element, the holder being located above the receptacle;
- it comprises a holder at least one an array of raw microneedles, the holder being located above the receptacle, the holder being located above the receptacle and is moveable in order to immerse the array of raw microneedles in the receptacle and in front of at least one conductive element located in the solution into the receptacle;
- the holder is moveable in order to immerse the array of raw microneedles into the receptacle.

The invention has several advantages.

The use of a conductive element well-disposed allows a deposit of a homogenous conductive layer or enzyme layer. Furthermore, the method and the system are suitable for a scale-up manufacturing.

### BRIEF DESCRIPTION OF THE DRAWINGS

Other features and advantages of the invention will appear in the following description. Embodiments of the invention will be described with reference to the drawings, in which:
- Figure 1 illustrates a view of the different elements of a body monitoring device using microneedles fabricated with the invention,
- Figure 2 illustrates a schematic view of a system for fabricating an array of microneedles according to a first embodiment,
- Figure 3 illustrates different steps of a method for fabricating microneedles according to an embodiment of the invention,
- Figures 4A and 4B illustrate a view of second and third embodiments of a system for fabricating several arrays of microneedles.

On all the figures, similar elements have identical references.

### DETAILED DESCRIPTION OF THE INVENTION

### Body monitoring device with sensors

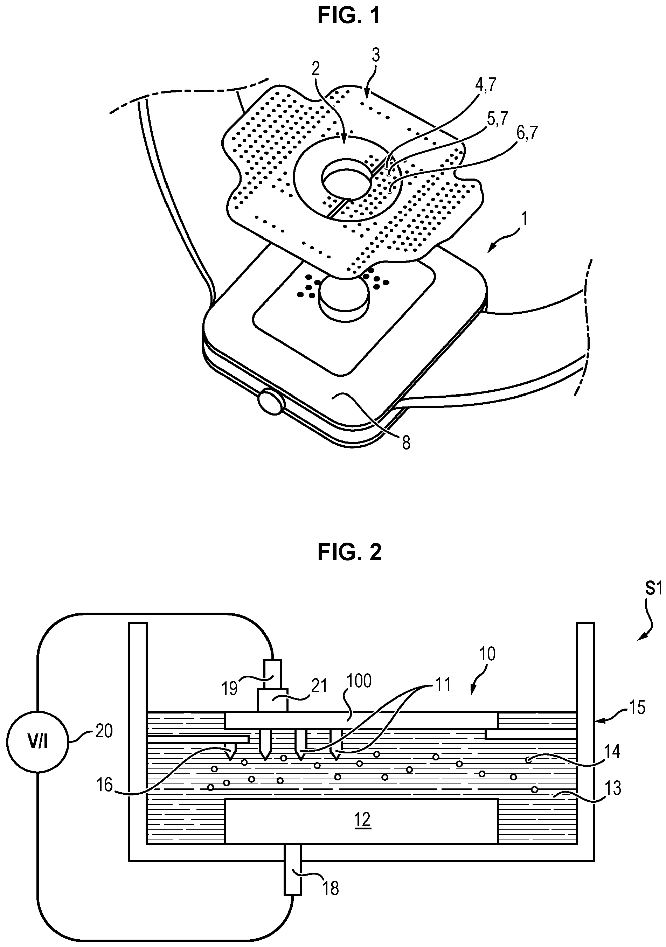

Figure 1 illustrates a device 1 for measuring/monitoring a body analyte in fluid of a user comprising a sensor 2 surrounded by a patch 3 including an adhesive layer for attaching the sensor 2 to the wearer's skin.

The sensor 2 comprises a working electrode 4, a counter electrode 5 and preferably a reference electrode 6. These electrodes are inserted in the dermis of a user by several microneedles 7 to as to be in contact with the interstitial fluid of the user. Each electrode is thus constituted by at least one microneedle 7. The sensor 2 is in particular an electrochemical sensor.

The sensor 2 preferably comprises between four and fifty microneedles 7 or even four hundred microneedles 7. Of course, a different number may be considered without limiting the scope of the description.

A microneedle 7 is defined as a needle with a low height preferably between 0.2 µm and 1 mm, preferably between 0.3 mm and 0.8 mm. The height of the microneedles 7 is low enough to avoid contact with a mechanical pain nerve of the wearer when the device 1 is worn.

The microneedles 7 are adapted to pierce the skin to contact the interstitial fluid when the sensor 2 is brought into contact with the skin.

The device 1 also comprises a control unit 8. The control unit 8 is electrically connected to the electrodes, particularly to the working electrode 4, to the counter electrode 5, and preferably to the reference electrode 6. The control unit 8 can comprise processor, a memory, an electrical acquisition module and an electrical control module. Preferably, every working electrode 4 and counter electrode 5 is independently electrically connected to the electrical acquisition module and to the electrical control module.

Each microneedle 7 comprises on its surface at least one electrically conductive layer and a biochemical element (such an enzyme) for the working electrode, the biochemical element being configured for oxidizing the analyte.

The conductive layer is for example platinum or silver or gold etc. The enzyme comprises a cofactor which is responsible for the oxidation of the analyte, preferably glucose or lactate. The enzyme is preferably an enzyme chosen between glucose oxidase and dehydrogenase, such as PQQ-glucose dehydrogenase, NAD-glucose dehydrogenase or FAD-glucose dehydrogenase or lactate oxidase, etc.

The electrically conductive layer is obtained during a metallization step and the enzyme is deposited during a functionalization step of a method for fabricating microneedle as it will be described in the following. The functionalization is in particular performed using electrodeposition concept.

### Metallization and/or functionalization of an array of microneedle

In the following we describe metallization and functionalization implemented for the fabrication of microneedles 7 intended to be used into an electrochemical sensor 2 as, for instance, described above.

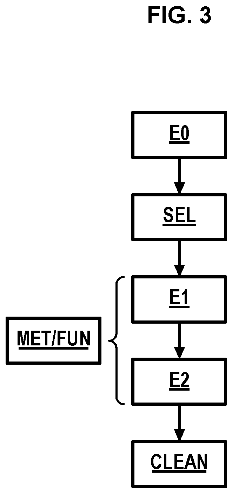

Figure 2 illustrates a system S1 for implementing the metallization or functionalization of raw microneedles 11 according to a first embodiment and figure 3 illustrates steps for implementing the metallization or functionalization.

In the following description, by "microneedle" 7 is meant a microneedle usable to be positioned on a sensor 2, and by "raw microneedle" 11, a microneedle which is a microneedle needing treatments in order to be ready to be used in the electromechanical sensor. A raw microneedle 11 has therefore a shape identical to the microneedle 7 used in the electromechanical sensor. Thus, depending on the context, the designated raw microneedle 11 can be a microneedle without any kind of treatment or a metallized microneedle just before the functionalization of such a metallized microneedle.

As illustrated, an array 10 of raw microneedles 11 is disposed in front of a conductive element 12. The conductive element 12 is, in particular, at equidistance of every raw microneedle 11 and not in contact with the tips of the raw microneedles 11 (step E0). Preferably, the tips of each raw microneedle 11 is in front of the conductive element 12, each at a same distance of the conductive element 12, the distance being, for instance, comprises between 0,1mm and 10mm. Preferably, the positioning accuracy must be greater than 90%, i.e., the distance between each tip of raw microneedle and the conductive element 12 must not be greater than 10% of this distance.

The array 10 of raw microneedles 11 and the conductive element 12 are immersed in a solution 13 comprising an active element 14 (step E1). The solution 13 is advantageously contained in a receptable 15 which is a bath or a tank or container able to receive a solution 13.

The array of raw microneedles 11 and the conductive element 12 are connected to a power source 20 configured to apply a potential V or a current I to the conductive 12 and the array of raw microneedles 11.

Then a potential V or a current I between the conductive element 12, forming a cathode, and at least one raw microneedle 11 of the array 10 of raw microneedles 11, forming an anode, is applied (step E2), causing an electrodeposition of a uniform and homogenous film on the raw microneedles 11, the film comprising the active element 14. In other words, the conductive element 12 and the array are power supplied.

Preferably, the potential V (or the current I) applied is an oxidizing potential V (or current I) and/or a reducing potential V (or current I), the potential V (or current I) being a fixed potential V (or current I) or a variable potential V (or current I). In addition, the potential V (or current I) is applied using, for instance, amperometric method and/or chronoamperometric method and/or potentiometric method and/or voltametric cycle. The potential (or current) is applied during a time that can be from a few minutes to a few hours. The amplitude of the potential V (or current I) which is applied can be from a few tens millivolt to a few hundred millivolt.

The cathode and the anode are subject to the potential V (or current I) to start electrodeposition applying uniform and homogenous film on the raw microneedles 11 on which the potential V (or current I) is applied. During the electrodeposition the film comes from active element 14 comprised in the solution 13. The amount of potential V (or current I) is linked to the thickness of the deposited film.

Advantageously, the solution 13 is a phosphate-buffered saline solution. The active element 14 comprised in the solution 13 is metallic particles or an electroactive monomer precursor. The solution 13 with metallic particles is used for the metallization step (MET) and the solution 13 with the enzyme is used for the functionalization step (FUN). The metallic particles are for instance made platinum or silver or gold, etc. The electroactive monomer precursor is for instance, Pyrrole, with a biochemical element 9, e.g., GOX or another enzyme as already described.

The conductive element 12 is preferably a grid with the axis of the holes parallel to the array 10 of raw microneedles 11. Optionally the conductive element 12 is a plate of any form or it can be a network of tips positioned in front of each raw microneedle 11. By tips it is mean everything that can protrude regularly or not from a surface and that extends in the same direction i.e., bosses, points, crenellations, curves.

The microneedles obtained with the method herein described can be used as different type of electrode and during the fabrication it is possible to select a group of raw microneedles 11 and to use the adequate solution 13, and then to apply the potential V (or current I) only on the selected raw microneedles 11. It has to be understood that only the raw microneedles 11 on which the potential V (or current I) is applied are metallized or functionalized.

Thus, the anode is defined by each raw microneedle 11 connected to the source 20.

Advantageously, in addition to the conductive element 12, an external reference electrode 16 is immersed and the potential V (or current I) is also applied to this external reference electrode 16. This external reference electrode 16 is in particular disposed not to be in contact with the raw microneedles 11 and the conductive element 12. The external reference electrode 16 is used to control and/or adapt the amplitude and/or the duration of the potential V (or current I). The conductive element 12 and the external reference electrode 16 are always subjected to the potential (or current) when present.

As illustrated in figure 3, once the array of raw microneedles 11 is disposed in front of the conductive element 12 (step E0), the method may comprise a selection of a group of at least one raw microneedle 11 (step SEL1) for defining an anode and then comprises the steps E1 and E2. Steps E1 and E2 define a metallization step (step MET) when the solution comprises metallic particles and define a functionalization step (step FUN) when the solution comprises the enzyme.

It can be noticed that the functionalizing step (step FUN) is preferentially processed on raw microneedles 11 which have been metallized before.

One can note that each raw microneedle 11 or each group of raw microneedles 11 can be connected independently so that each raw microneedle 11 can be fabricated independently. Thus, it is possible to functionalize some raw microneedles 11 without functionalizing the other.

The metallization and the functionalization can be implemented successively without removing the array 10 of raw microneedles 11 from the receptacle 15. Each deposit is performed on the raw microneedles 11 which are connected to the power source 20. Preferably, it is possible to subject independently each raw microneedle 11 or group of raw microneedles 11 to the potential V (or current I), and then to turn on power for only some raw microneedles 11 of the array 10 of raw microneedles 11.

As illustrated on figure 2, the system S1 according to the first embodiment is such that the conductive element 12 is disposed in the receptacle 15 and the array 10 of raw microneedles 11 is disposed above the conductive element 12. Also, this system S1 comprises a first electrical connector 18 to connect the conductive element 12 to the power source 20, a second electrical connector 19 connected to the array 10 of raw microneedles 11 to the power source 20. If needed, the external reference electrode 16 is also connected to the power source 20.

Advantageously, the system S1 includes a seal 21 to isolate the electrical connector 19 from the solution 13 when the array 10 of raw microneedles 11 is immersed. The array 10 of raw microneedles 11 comprises a plate 100 from which the raw microneedles 11 extend.

The array 10 of raw microneedles 11 is immersed so that the raw microneedles 11 are parallel to the direction of immersion and the electrical connector 19 stays out of the solution 13 and is isolated from it by the seal 21.

In a complementary way, the first embodiment can be used to fabricate several arrays 10 at the same time by disposing several arrays 10 of raw microneedles 11 above the conductive element 12.

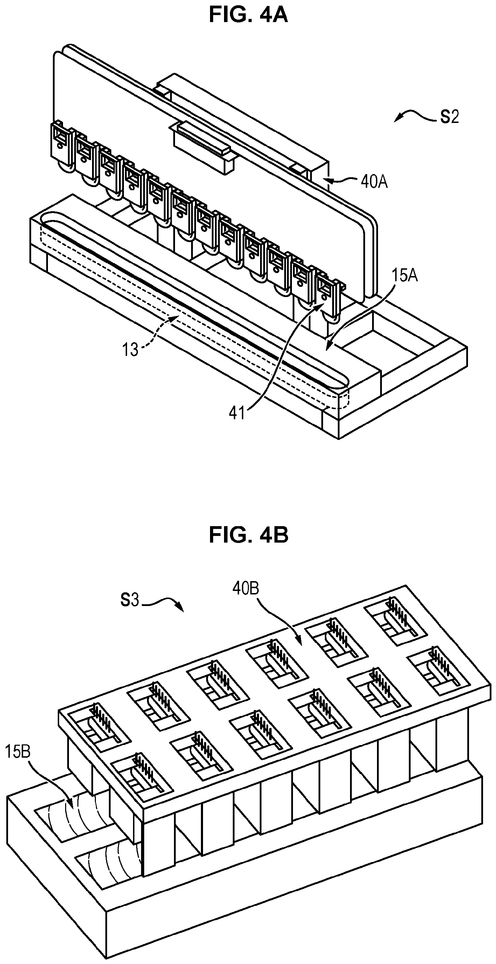

Figure 4A, illustrates a system S2 for fabricating microneedles 7 according to a second embodiment. This system S2 comprises a holder 40A for holding several units 41 each comprising a conductive element 12 and an array 10 of raw microneedles 11. The holder 40A is positioned above a receptable 15A configured to be filled with the solution 13 and is moveable to immerse each unit 41 into the solution 13.

Figure 4B illustrates a system S3 for fabricating microneedles 7 according to a third embodiment. This system S3 comprises a holder 40A comprising two parallel channels defining a receptacle 15A configured to be filled with the solution 13. Here again, each unit 41 comprises a conductive element 12 and an array 10 of raw microneedles 11. The holder 40B is positioned above a receptable 15B configured to be filled with the solution 13 and is moveable to immerse each unit 41 into the solution 13.

By having the array and the conductive element 12 together facilitate the changing of one of them.

The second and third embodiments permit to fabricate several arrays at the same time. Also, contrary to the first embodiment the conductive element 12 and the array are immersed so that the main axis of each microneedle is perpendicular to the direction of immersion. Moreover, the holder 40A can facilitate the installation and/or the removal of each array of microneedles 3 on the system S2, S3.

Moreover, and according to one embodiment, the holder 40A, 40B of each system S2 or S3 supports at least one an array 10 of raw microneedles 11 only, the conductive element 12 being in the receptable 15, 15. Therefore, when the array(s) 10 of raw microneedles 11 is immersed, it is in front of at least one conductive element 12. Thus, only the array of raw microneedles 11 is changed not the conductive element.

## Claims

1. A method comprising the steps of:
a) disposing (E0) an array (10) of raw microneedles (11) in front of a conductive element (12) so that the conductive element (12) is at equidistance of every raw microneedle (11) and not in contact with the tips of the raw microneedles (11);
b) immersing (E1) the array (10) of raw microneedle(s) (11) and the conductive element (12) in a solution (13) comprising an active element (14),
c) applying (E2) a potential (V) or a current (I) between the conductive element (12), forming a cathode, and at least one raw microneedle (11) of the array (10) of raw microneedles (11) forming an anode, causing an electrodeposition of a uniform and homogenous film comprising the active element (14) on the raw microneedles (11) of the anode.

2. The method as claimed in claim 1, wherein an external reference electrode (16) is disposed into the solution (13) and not to be in contact with the raw microneedles (11) and the conductive element (12), and not between the raw microneedles (11) and the conductive element (12), the potential (V) being also applied to the external reference electrode (16).

3. The method as claimed in any one of claims 1 to 2, wherein the conductive element (12) is a conductive grid or mesh plate or array of tips.

4. The method as claimed in any one of claims 1 to 3, wherein the solution (13) is a saline solution, the active element (14) being metallic particles or an electroactive monomer precursor with a biochemical element.

5. The method as claimed any one of claims 1 to 4, wherein the tip of each raw microneedle (11) is in front of the conductive element (12), each at a same distance of the conductive element (12), the distance being comprise between 0,1mm and 10mm.

6. The method as claimed in any one of claims 1 to 5, for use as a metallization step (MET) wherein the active element (14) is conductive particles, the microneedles obtained at step c) are metallized microneedles (11) adapted to be an electrode (7) for an electrochemical sensor (2).

7. The method as claimed in any of claims 1 to 6, for use as a functionalizing step (FUN) wherein the active element (14) is an electroactive monomer precursor with a biochemical element, the microneedles obtained at step c) are functionalized microneedles (11") adapted to be used in a working electrode (4) for an electrochemical sensor (2).

8. The method as claimed in any of claims 1 to 7, wherein the potential (V) or current (I) is an oxidizing potential or current and/or a reducing potential or current, the potential (V) or current being a fixed potential or a variable potential said potential (V) or current (I) being applied (E2) using amperometric method and/or chronoamperometric method and/or potentiometric method and/or chronometric method and/or voltametric cycle.

9. System (S1, S2, S3) comprising:
a receptacle (15A, 15B) configured to be filled with a solution (13) comprising an active element (14),
at least one conductive element (12) intended to be disposed in front of an array (10) of raw microneedles (11) and at equidistance of every raw microneedle (11) and not in contact with the tips of the raw microneedles (11),
a power source (20) configured to apply (E2) a potential (V) or a current (I) between the conductive element (12), forming a cathode, and at least one raw microneedle (11) of an array (10) of raw microneedles (11), forming an anode, the conductive element (12) aiming at an electrodeposition of a uniform and homogenous film on each the raw microneedles (11) of the anode when immersed in the solution (13) and when the potential (V) is applied, the film comprising the active element (14).

10. System (S2, S3) according to claim 9, comprising a holder (40A, 40B) for holding at least one conductive element (12) and at least one an array (10) of raw microneedles (11) in front of the conductive element (12), the holder (40A, 40B) being located above the receptacle (15A, 15B).

11. System (S2, S3) according to claim 9, comprising a holder (40A, 40B) at least one an array (10) of raw microneedles (11), the holder (40A) being located above the receptacle (15A, 15B), the holder (13) being located above the receptacle (1) and is moveable in order to immerse the array (10) of raw microneedles (11) in the receptacle (15A) and in front of at least one conductive element (12) located in the solution (13) into the receptacle (15A, 15B).

12. System (S2, S3) according to anyone of claims 9 to 11, wherein the holder (40A, 40B) is moveable in order to immerse the array (10) of raw microneedles (11) into the receptacle (15A, 15B).
